# EUROPÄISCHE PATENTSCHRIFT

(11) **EP 2 060 847 B1**
(45) Veröffentlichungstag und Bekanntmachung des Hinweises auf die Patenterteilung: **20.07.2016**
(21) Anmeldenummer: 07022199.9
(22) Anmeldetag: 15.11.2007
(51) Int. Cl.: F21S 8/00

(54) **Operationsleuchte**
Operating light
Lampe chirurgicale

(43) Veröffentlichungstag der Anmeldung: 20.05.2009
(73) Patentinhaber: Frowein EZH GmbH, 42117 Wuppertal (DE)
(72) Erfinder: Diez, Ingolf, 78532 Tuttlingen (DE)
(74) Vertreter: Feder Walter Ebert

(56) Entgegenhaltungen:
- EP-A- 1 568 934
- EP-A- 1 722 157
- EP-A- 1 750 052
- WO-A-2007/036581

## Beschreibung

Die Erfindung betrifft eine Operationsleuchte gemäß den Merkmalen des Oberbegriffs des Patentanspruchs 1. Eine solche Operationsleute ist aus der EP-A-1 722 157 bekannt. Dort werden wahlweise einzelne Module der Operationsleuchte eingeschaltet oder ausgeschaltet, wobei sich das beleuchtete Feld nicht ändert, um Schattenwurf zu vermeiden.

Die Bezeichnung "Operationsleuchte" betrifft eine Leuchte, die für medizinische Anwendungen geeignet ist. Die erfindungsgemäße Operationsleuchte ist insbesondere zur Beleuchtung eines Operationsfeldes, beispielsweise bei einem chirurgischen Eingriff geeignet. Die Operationsleuchte kann beispielsweise auch zur Beleuchtung eines Mundinnenraums (Dentalleuchte) eingesetzt werden. Solche Operationsleuchten dienen auch zu Diagnosezwecken und zu Behandlungen im Bereich der Veterinärmedizin.

Aus dem Stand der Technik sind Operationsleuchten mit mehreren Lichtquellen und mehreren den Lichtquellen zugeordneten optischen Abbildungsvorrichtungen bekannt.

Bei der Verwendung von Operationsleuchten ist es oft notwendig, das von der Operationsleuchte erzeugte Lichtfeld zu verändern. Als Lichtfeld wird der auf dem Operationsfeld abgebildete Ausleuchtungsbereich bezeichnet. Beispielsweise wird in der Chirurgie bei größeren invasiven Eingriffen ein weiteres Lichtfeld benötigt. Dazu wird beispielsweise bei runden Lichtfeldern das Lichtfeld auf dem Operationsfeld von 160 mm Durchmesser auf 260 mm Durchmesser ausgeweitet. Eine solche Ausweitung des Lichtfelds führt jedoch zu einer Verringerung der Beleuchtungsstärke auf dem Operationsfeld. Dies kann soweit führen, dass die Beleuchtungsstärke auf dem Operationsfeld für die Durchführung von chirurgischen Eingriffen zu gering ist.
Darüber hinaus gibt es auch Anwendungsfälle für Operationsleuchten, bei denen das Lichtfeld auf dem Operationsfeld verringert werden muss. Beispielsweise wird bei der minimal-invasiven Chirurgie nur ein konzentrierter chirurgischer Eingriff an einem Körper vorgenommen. Entsprechend ist nur eine Ausleuchtung des relativ kleinen chirurgischen Eingriffsbereichs notwendig. Bei einer solchen Verkleinerung des Lichtfelds kann sich jedoch das Problem ergeben, dass die Beleuchtungsstärke auf dem Operationsfeld zu hoch wird, da die Strahlung der Lichtquellen von einem weiten Lichtfeld auf ein kleineres Lichtfeld reduziert wird.
Bei bekannten Operationsleuchten verändert sich folglich die Beleuchtungsstärke auf dem Operationsfeld in Abhängigkeit von der Größe des Lichtfelds. Eine solche Änderung der Beleuchtungsstärke ist jedoch unerwünscht, da dadurch die Anwendung auf dem Operationsfeld, beispielsweise ein chirurgischer Eingriff, gestört werden kann.

Bei bekannten Operationsleuchten erfolgt die Verstellung des Lichtfelds auf dem Operationsfeld über eine mechanische oder eine elektromechanische Verstellung der Lichtquellen in einem Reflektor und/oder durch eine mechanische oder elektromechanische Verschwenkung eines oder mehrere Reflektoren. Solche mechanischen bzw. elektromechanischen Verstell- bzw. Verschwenkvorrichtungen sind jedoch konstruktiv aufwendig und führen zu erhöhten Kosten der Operationsleuchte. Darüber hinaus unterliegen solche mechanischen bzw. elektromechanischen Konstruktionen einer gewissen Störanfälligkeit.

Aus der Druckschrift US 6,880,957 B2 ist ferner eine Operationsleuchte bekannt, bei der zur Verhinderung von durch zwischen einer Leuchte und dem Operationsfeld befindlichen Objekten auf dem Operationsfeld erzeugten Schatten, in Abhängigkeit von den Objekten die Strahlungsleistung einzelner Strahler der Operationsleuchte gesteuert werden kann. In einer Ausführungsform werden zur Verhinderung von Schatten auf dem Operationsfeld nur fünf von zehn Lichtquellen genutzt. Es erfolgt jedoch keine Steuerung der Strahlungsleistung der verbliebenen Lichtquellen in Abhängigkeit von den nicht genutzten Lichtquellen. Darüber hinaus erfolgt durch die Reduzierung der Anzahl von Lichtquellen keine Verringerung des Leuchtfeldes der Operationslampe.

Aufgabe der Erfindung ist es, eine Operationsleuchte bereitzustellen, welche dem Operateur einen erhöhten Bedienungs- und Arbeitskomfort bietet.

Die hierzu vorgesehene erfindungsgemäße Operationsleuchte ist im Patentanspruch 1 beschrieben.

Die abhängigen Patentansprüche beschreiben bevorzugte Ausgestaltungen der Operationsleuchte.

Bevorzugt handelt es sich bei der Mehrzahl von Lichtquellen um Leuchtdioden (LEDs). LEDs haben gegenüber Glühlampen den Vorteil, dass sie keine Temperaturstrahler sind und Licht nur in einem begrenzten Spektralbereich emittieren. Bevorzugt erzeugen die LEDs ein weißes Licht mit einer hohen Farbtemperatur. Dadurch ergibt sich ein natürlicher Farbeindruck auf dem Operationsfeld und ein ermüdungsfreies Arbeiten mit der Operationsleuchte wird ermöglicht.

LEDs weisen eine hohe Lichtausbeute auf. Der Erfindung liegt insbesondere die Erkenntnis zugrunde, dass LEDs auch eine zu hohe Lichtausbeute für eine Anwendung in einer Operationsleuchte aufweisen können. Beispielsweise haben heutzutage auf dem Markt erhältliche LEDs eine Lichtausbeute von 60 Im/W. Zukünftig auf dem Markt erhältliche LEDs werden schon eine Lichtausbeute von ungefähr 80 Lm/W aufweisen. Gemäß der internationalen Norm IEC 60601-2-41 ist die maximal zulässige Beleuchtungsstärke jedoch auf 160 klx begrenzt. Somit besteht insbesondere bei der Verwendung von LEDs als Lichtquellen das Problem, dass eine zu hohe Beleuchtungsstärke auf dem Operationsfeld erzeugt werden kann. Dieses Problem ergibt sich insbesondere dann, wenn das Lichtfeld auf dem Operationsfeld verringert wird. Dabei besteht die Gefahr, dass eine zu hohe Beleuchtungsstärke auf dem Operationsfeld erzeugt wird.

Bei den optischen Abbildungsvorrichtungen handelt es sich bevorzugt um Reflektorelemente. Mit Hilfe der optischen Abbildungsvorrichtungen können eine Vielzahl übereinandergelagerter Lichtfelder erzeugt werden, was zu einem homogenen Licht führt. Bei den Reflektorelementen kann es sich um Polygonreflektoren handeln.

Erfindungsgemäß sind Mittel zum Abschalten mindestens einer Lichtquelle aus der Mehrzahl von Lichtquellen vorgesehen. Mit Hilfe der Mittel zum Abschalten kann die Beleuchtungsstärke auf dem Operationsfeld verringert werden. Zusätzlich sind Mittel zum Steuern der Strahlungsleistung mindestens einer anderen Lichtquelle aus der Mehrzahl von Lichtquellen vorgesehen, welche in Abhängigkeit von dem Abschalten der mindestens einen Lichtquelle mindestens eine andere Lichtquelle aus der Mehrzahl von Lichtquellen steuern. Bei den Mitteln zum Abschalten und den Mitteln zum Steuern der Strahlungsleistung kann es sich beispielsweise um einen Mikrocontroller oder eine elektrische Steuerungsschaltung handeln, welche mit den Lichtquellen verbunden ist, und diese steuert. Somit kann die Beleuchtungsstärke mit Hilfe der Mittel zum Steuern der Strahlungsleistung auf den gewünschten Wert geregelt werden. Die Regelung kann beispielsweise mit einem Pulsweitenmodulationsverfahren oder einem anderen passenden Regelungsverfahren erfolgen.

Die Mittel zum Steuern sind dazu eingerichtet, die Strahlungsleistung der mindestens einen anderen Lichtquelle der Mehrzahl von Lichtquellen so zu steuern, dass die Beleuchtungsstärke auf dem Operationsfeld konstant bleibt. Auch wenn einzelne der Mehrzahl von Lichtquellen abgeschaltet werden, so soll dennoch die Beleuchtungsstärke auf dem Operationsfeld konstant bleiben. Es ist dabei nicht gewünscht, dass durch das Abschalten von Lichtquellen die Beleuchtungsstärke auf dem Operationsfeld unter einen vorgegebenen Mindestwert sinkt. Beispielsweise ist es bei der minimal-invasiven Chirurgie, bei der sehr präzise gearbeitet werden muss, notwendig, dass eine Veränderung der Beleuchtungsstärke auf dem Operationsfeld vermieden wird, so dass der chirurgische Eingriff durch die Operationsleuchte nicht beeinflusst wird. Eine zu geringe Beleuchtungsstärke kann beispielsweise auch dann Probleme bereiten, wenn zu operierendes Körpergewebe nur schwer zugänglich ist und eine direkte Beleuchtung des Gewebes nicht möglich ist.

Durch das Abschalten der mindestens einen der Mehrzahl von Lichtquellen kann das Lichtfeld auf dem Operationsfeld verringert werden. Das Lichtfeld wird dabei verringert, um es den Erfordernissen des Operation anzupassen. Eine Verringerung des Lichtfeldes erfolgt aber nicht, um Schatten durch zwischen der Leuchte und dem Operationsfeld befindliche Objekte zu vermeiden. Will man beispielsweise bei der minimal-invasiven Chirurgie ein weites Lichtfeld auf dem Operationsfeld auf ein konzentriertes Lichtfeld verringern, so ist es möglich, jeweilige Lichtquellen gezielt auszuschalten. So können beispielsweise Lichtquellen ausgeschaltet werden, welche einen äußeren Teil des Lichtfelds ausleuchten. Denkbar ist aber auch, dass Lichtquellen ausgeschaltet werden, welche einen anderen Bereich, beispielsweise einen Innenbereich, des Lichtfelds ausleuchten. Durch dieses Abschalten von Lichtquellen wird jedoch die Beleuchtungsstärke des verkleinerten Lichtfelds auf dem Operationsfeld reduziert. Diese Verringerung der Beleuchtungsstärke kann dadurch begründet sein, da die abgeschalteten Lichtquellen auch das verkleinerte Lichtfeld beleuchtet haben. Beispielsweise haben vor dem Abschalten von Lichtquellen 30 Lichtquellen zum Erzeugen des Lichtfelds beigetragen, wohingegen nach dem Abschalten von Lichtquellen nur noch 18 Lichtquellen zum Erzeugen des Lichtfelds beigetragen. Eine solche Verringerung der Beleuchtungsstärke ist jedoch unerwünscht, da diese beispielsweise die Durchführung von medizinischen Behandlungen beeinträchtigen kann. Erfindungsgemäß kann mit Hilfe der Mittel zum Steuern der Strahlungsleistung jedoch mindestens eine der eingeschalteten Lichtquellen so gesteuert werden, dass die Beleuchtungsstärke auf dem Operationsfeld, auch bei Abschalten einzelner Lichtquellen konstant bzw. nahezu konstant bleibt. Die Steuerung der Beleuchtungsstärke kann auch derart erfolgen, dass sie nicht unter einen vorgegebenen Mindestwert fällt und/oder einen vorgegebenen Höchstwert nicht überschreitet.

Bevorzugt kann durch das Abschalten der mindestens einen der Mehrzahl von Lichtquellen das Lichtfeld auf dem Operationsfeld von einer Kreisfläche mit einem ersten Durchmesser auf eine Kreisfläche mit einem zweiten Durchmesser verändert werden, wobei der zweite Durchmesser kleiner als der erste Durchmesser ist. Gemäß dieser Ausführungsform wird die Kreisfläche, d.h. der Durchmesser, des Lichtfelds verringert und keine Verringerung des Lichtfelds erfolgt dadurch, dass eine einzelne Bereiche des Lichtfelds nicht beleuchtet werden.

Gemäß einer Weiterbildung der Erfindung sind die Mittel zum Steuern dazu eingerichtet, die Strahlungsleistung aller anderen der Mehrzahl von Lichtquellen so zu steuern, dass die Beleuchtungsstärke auf dem Operationsfeld konstant bleibt. Dadurch wird eine noch einfachere Steuerung der Beleuchtungsstärke ermöglicht. Auch hier kann die Steuerung der Beleuchtungsstärke derart erfolgen, dass sie nicht unter einen vorgegebenen Mindestwert fällt und/oder Höchstwert überschreitet.

Bei der Operationsleuchte gemäß der vorliegenden Erfindung ist es nicht notwendig, dass die Lichtquellen und/oder die optischen Abbildungsvorrichtungen dazu eingerichtet sind, zur Veränderung des Lichtfelds auf dem Operationsfeld mechanisch und/oder elektromechanisch verstellt zu werden. Gemäß der vorliegenden Erfindung kann das Lichtfeld durch Ein- bzw. Abschalten von Lichtquellen verändert werden und die Beleuchtungsstärke in dem veränderten Lichtfeld kann mit Hilfe der Mittel zum Steuern der Strahlungsleistung, welche die Strahlungsleistung der Lichtquellen steuern, verändert bzw. so gesteuert werden, dass die Beleuchtungsstärke auf dem Operationsfeld auch bei Veränderung des Lichtfelds konstant bleibt. Mechanische bzw. elektromechanische Verstell- bzw. Verschwenkvorrichtungen für die Lichtquellen und/oder die optischen Abbildungsvorrichtungen erfordern einen hohen konstruktiven Aufwand hinsichtlich der mechanischen Ausgestaltung und der Steuerungselektronik. Eine Vermeidung solcher mechanischen bzw. elektromechanischen Vorrichtungen hat den Vorteil, dass die Operationsleuchte einfacher und mit weniger Bauteilen gebaut werden kann. Dadurch wird die Produktionszeit der Operationsleuchte und auch deren Kosten reduziert. Darüber hinaus wird durch die Vermeidung einer solchen mechanischen bzw. elektromechanischen Konstruktion auch die Störanfälligkeit der Operationsleuchte verringert, was die durchschnittliche Lebenszeit der Operationsleuchte erhöht. Es ist aber auch denkbar, dass die Operationsleuchte Verstell- bzw. Verschwenkvorrichtungen für die Lichtquellen und/oder die optischen Abbildungsvorrichtungen aufweist, diese aber für die Veränderung des Lichtfeldes und die Steuerung der Beleuchtungsstärke nicht bzw. in einem bestimmten Betriebsmodus der Operationsleuchte nicht verwendet werden.

Die Mittel zum Abschalten mindestens einer der Mehrzahl von Lichtquellen können auch dazu eingerichtet sein, jede der Lichtquellen unabhängig voneinander abzuschalten. Durch entsprechende Anordnung der Lichtquellen bzw. der optischen Abbildungsvorrichtungen ist es möglich, beliebige Bereiche auf dem Operationsfeld mit einem variablen Lichtfeld bei gewünschter Beleuchtungsstärke auf dem Operationsfeld auszuleuchten. Gemäß dieser Ausführungsform ist es beispielsweise möglich, zwischen verschiedenen Lichtfeldern auf dem Operationsfeld bzw. zwischen weiten und konzentrierten Lichtfeldern zu wechseln, ohne dass sich die Beleuchtungsstärke ändert.

Gemäß einer Weiterbildung der Erfindung sind die optischen Abbildungsvorrichtungen in der Operationsleuchte so angeordnet, dass sie etwa eine Kreisfläche bilden. Die Lichtquellen und optischen Abbildungsvorrichtungen können auch als separate Leuchten ausgebildet sind. Beispielsweise kann eine Leuchte drei Lichtquellen und drei optische Abbildungsvorrichtungen auweisen. Entsprechend können auch mehrere Leuchten so angeordnet sein, dass sie etwa eine Kreisfläche bilden. Die Erfindung ist jedoch nicht auf Kreisflächen beschränkt. Grundsätzlich können die Abbildungsvorrichtungen bzw. Leuchten in jeder beliebigen Form angeordnet sein.

Die Mittel zum Abschalten mindestens einer der Mehrzahl von Lichtquellen können dazu eingerichtet sein, die am äußeren Rand der Kreisfläche angeordneten Lichtquellen abzuschalten. Entsprechend können auch die Mittel zum Abschalten mindestens einer der Mehrzahl von Lichtquellen dazu eingerichtet, die am äußeren Rand der Kreisfläche angeordneten Leuchten abzuschalten. Gemäß dieser Ausführungsform wird ein kreisrundes Lichtfeld auf dem Operationsfeld abgebildet. Durch Abschalten der am äußeren Rand der Kreisfläche angeordneten Lichtquellen bzw. Leuchten wird das Lichtfeld auf eine verringerte Kreisfläche auf dem Operationsfeld reduziert. Falls keine Erhöhung der Strahlungsleistung der verbliebenen strahlenden Lichtquellen bzw. Leuchten stattfinden würde, so würde sich die Beleuchtungsstärke des verringerten Lichtfeld reduzieren. Eine solche reduzierte Beleuchtungsstärke könnte beispielsweise für einen minimal-invasiven chirurgischen Eingriff zu gering sein. Aus diesem Grund wird mit Hilfe der Mittel zum Steuern der Strahlungsleistung die Strahlungsleistung der verbliebenen noch strahlenden Lichtquellen bzw. Leuchten so gesteuert, dass die Beleuchtungsstärke auf dem Operationsfeld konstant bzw. nahezu konstant bleibt. Dabei ist es auch denkbar, dass nicht alle verbliebenen strahlenden Lichtquellen bzw. Leuchten gesteuert werden, sondern nur einzelne der verbliebenen strahlenden Lichtquellen bzw. Leuchten.

Die Mittel zum Steuern der Strahlungsleistung können auch dazu eingerichtet sein, die Beleuchtungsstärke auf dem Operationsfeld zu dimmen. Dadurch hat der Nutzer der Operationsleuchte zusätzlich die Möglichkeit, die Beleuchtungsstärke individuell anzupassen. Die Mittel zum Steuern der Strahlungsleistung können dabei so eingerichtet sein, dass sie bei dem Dimmen auch eine reduzierte Anzahl von strahlenden Lichtquellen berücksichtigen.

Bevorzugt ist die Operationsleuchte so ausgebildet, dass die Beleuchtungsstärke auf dem Operationsfeld 160 klx nicht übersteigt. Dadurch wird gewährleistet, dass keine zu hohe Beleuchtungsstärke auf dem Operationsfeld erzeugt wird. Bevorzugt wird die Einhaltung dieses Grenzwertes von den Mitteln zum Steuern der Strahlungsleistung gesteuert.

Die Erfindung wird im Anschluss anhand der beigefügten Zeichnungen weiter erläutert. Es stellen dar:
- Fig. 1: eine schematische Darstellung eines Ausführungsbeispiels einer erfindungsgemäßen Operationsleuchte, und
- Fig. 2: ein Ausführungsbeispiel einer Leuchte, welche in der Operationsleuchte gemäß Fig. 1 verwendet werden kann.

In den Figuren sind gleiche Elemente mit den gleichen Bezugszeichen gekennzeichnet.

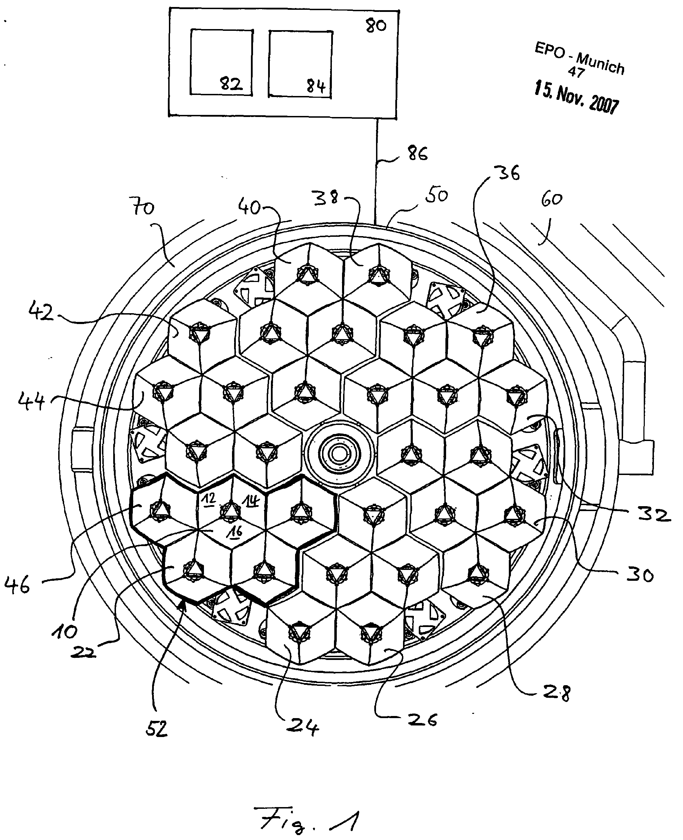

Fig. 1 zeigt in schematischer Darstellung eine Operationsleuchte in einer Ansicht von unten zusammen mit einer Steuerungsvorrichtung 80. Ein Gehäuse 50 der Operationsleuchte ist über eine Aufhängung 60 an der Decke (nicht gezeigt) eines Operationssaals befestigt. An dem Gehäuse 50 ist eine Griffung 70 befestigt, mit deren Hilfe das Gehäuse 50 durch einen Nutzer der Operationsleuchte in alle Richtungen bewegt werden kann. In dem Gehäuse 50 sind dreißig Leuchten in Form einer Kreisfläche angeordnet. In der Mitte der Leuchten befindet sich ein Griff.

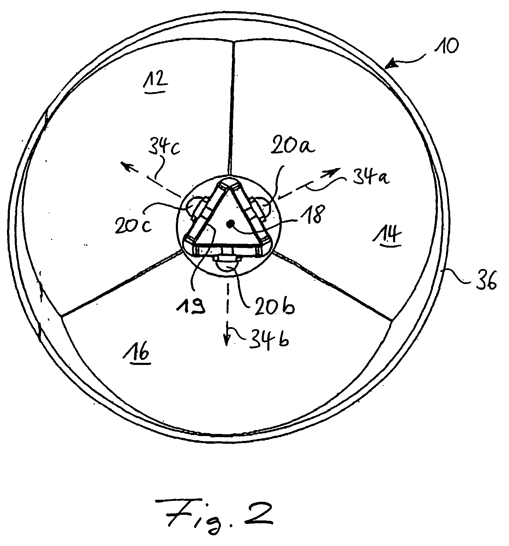

Der prinzipielle Aufbau einer Leuchte 10 wird unter Bezugnahme auf Fig. 2 beschrieben. Die Leuchte 10 weist ein Gehäuse 36 auf. In dem Gehäuse 36 sind drei Reflektorelemente 12, 14, 16 befestigt. An einem Sockel 19, der ebenfalls an dem Gehäuse 36 befestigt ist, sind drei LEDs 20a, 20b, 20c befestigt. Mit dem Bezugszeichen 18 ist die optische Achse der Leuchte 10 gekennzeichnet. Die LEDs 20a, 20b und 20c strahlen auf die ihnen zugeordneten Reflektorelemente 12, 14 und 16 und weisen Hauptstrahlungsrichtungen 34a, 34b und 34c auf. Die von den LEDs 20a, 20b und 20c emittierte Strahlung wird von den Reflektorelementen 12, 14, 16 reflektiert und auf ein Operationsfeld (nicht gezeigt) abgebildet. In dem gezeigten Ausführungsbeispiel sind die Reflektorelemente 12, 14, 16 fest an dem Gehäuse 36 befestigt. Dies bedeutet, die Reflektorelemente 12, 14, 16 sind nicht mechanisch oder elektromechanisch verstellbar. Darüber hinaus sind die LEDs 20a, 20b und 20c fest an dem Sockel 19 befestigt. Dies bedeutet ebenfalls, dass die LEDs 20a, 20b und 20c nicht mechanisch oder elektromechanisch verstellbar ausgebildet sind.

Die weiteren in Fig. 1 gezeigten Leuchten sind identisch zu der in Fig. 2 beschriebenen Leuchte 10 ausgebildet. Jeweils fünf Leuchten sind zu einer Leuchtenbaugruppe 52 zusammengefasst. Hinter den Leuchten befinden sich Anschlusselemente, die in Figur 1 nur teilweise sichtbar sind. Sämtliche in Fig. 1 gezeigte Leuchten sind fest in dem Gehäuse 50 angebracht. Dies bedeutet, dass die Leuchten nicht mechanisch oder elektromechanisch verstellbar sind.

Die Steuerungseinheit 80 ist als ein Mikrocontroller ausgebildet. Die Steuerungseinheit 80 umfasst eine Vorrichtung 82 zum Ein- und Abschalten einzelner Leuchten und eine Vorrichtung 84 zum Steuern der Strahlungsleistung der LEDs. Die Vorrichtung 82 ist so ausgebildet, dass sie sowohl einzelne Leuchten als auch einzelne LEDs ein bzw. Abschalten kann. Darüber hinaus kann die Vorrichtung 82 auch die Strahlungsleistung einzelner Leuchten und einzelner LEDs separat steuern. Die Steuerung der Strahlungsleistung der LEDs kann beispielsweise mit einem Pulsweitenmodulationsverfahren erfolgen.

In dem vorliegenden Ausführungsbeispiel ergibt sich, falls alle Leuchten eingeschaltet sind, ein rundes Lichtfeld auf dem Operationsfeld mit einem Durchmesser von 300 mm. Ein solches Lichtfeld mit einem Durchmesser von 300 mm kann jedoch für einen minimal-invasiven chirurgischen Eingriff zu groß sein. Aus diesem Grund kann ein Nutzer der Operationsleuchte einen Kontrollschalter oder einen Regler (nicht gezeigt) betätigt, welcher die Vorrichtung 82 dazu veranlasst zwölf Leuchten, d.h. die Leuchten 22, 24, 26, 28, 30, 32, 36, 38, 40, 42, 44 und 46, abzuschalten. Durch diese Abschaltung von zwölf Leuchten reduziert sich das Lichtfeld auf dem Operationsfeld von 300 mm Durchmesser auf 200 mm Durchmesser. Die Beleuchtungsstärke bei dem Durchmesser von 300 mm betrug 160 klx. Bedingt durch das Abschalten der äußeren Leuchten 22, 24, 26, 28, 30, 32, 36, 38, 40, 42, 44 und 46 würde sich jedoch die Beleuchtungsstärke in dem verringerten Lichtfeld von 200 mm Durchmesser reduzieren, da die Leuchten 22, 24, 26, 28, 30, 32, 36, 38, 40, 42, 44 und 46 auch zum dem Ausleuchten des inneren Bereichs beigetragen haben. Dies würde dazu führen, dass für den minimal-invasiven chirurgischen Eingriff nicht mehr ausreichend Beleuchtungsstärke zur Verfügung steht. Aus diesem Grund regelt die Vorrichtung 84 zum Steuern der Strahlungsleistung die Strahlungsleistung der verbliebenen eingeschalteten Leuchten so nach, dass die Beleuchtungsstärke von 160 klx auch nach Abschalten der Leuchten 22, 24, 26, 28, 30, 32, 36, 38, 40, 42, und 44 konstant bleibt. Dadurch wird eine Beeinträchtigung des chirurgischen Eingriffs durch eine Veränderung bzw. Veränderung der Beleuchtungsstärke vermieden.

Folglich wird durch die vorliegende Erfindung eine Operationsleuchte bereitgestellt, welche mechanische bzw. elektromechanische Verstell- bzw. Verschwenkvorrichtungen zum Verändern des Lichtfelds der Operationslampe vermeidet, wobei dennoch eine gewünschte Veränderung des Lichtfelds bei konstanter bzw. ausreichender Beleuchtungsstärke ermöglicht wird. Darüber hinaus wird bei der Operationsleuchte gemäß der vorliegenden Erfindung auch dem Problem Rechnung getragen, dass beim Einsatz von LEDs eine zu Hohe Beleuchtungsstärke vorhanden sein kann, welche erst auf einen gewünschte Anwendungsbeleuchtungsstärke reduziert werden muss. Gemäß der vorliegenden Erfindung kann ein Chirurg auch bei einem veränderten Lichtfeld Konturen, Farben und Bewegungen auf dem Operationsfeld genau erkennen. Darüber hinaus können die Lichtverhältnisse genau an die individuellen Operationsbedingungen angepasst werden, was eine optimale Sehschärfe ermöglicht.

## Patentansprüche

1. Operationsleuchte mit
einer Anzahl von Leuchten (10),
den Leuchten (10) zugeordneten optischen Abbildungsvorrichtungen (12, 14, 16), welche dazu eingerichtet sind, von den Leuchten (10) emittierte Strahlung auf ein Operationsfeld zu richten und dort ein ausgeleuchtetes Lichtfeld zu erzeugen, und mit
Mitteln (82) zum Abschalten mindestens einer der Leuchten (10),
**dadurch gekennzeichnet, dass**
durch das Abschalten der mindestens einen Leuchte der Durchmesser des ausgeleuchteten Lichtfeldes verkleinert wird, wobei eine Vorrichtung (34) eingerichtet ist, in Abhängigkeit von dem Abschalten der mindestens einen Leuchte die Strahlungsleistung der nicht abgeschalteten Leuchten (10) so nachzuregeln, dass die Beleuchtungsstärke in dem Lichtfeld konstant bleibt.

2. Operationsleuchte nach Anspruch 1, wobei die Mittel zum Steuern (84) dazu eingerichtet sind, die Strahlungsleistung aller nicht abgeschalteten Leuchten (10) so zu steuern, dass die Beleuchtungsstärke in dem Lichtfeld konstant bleibt.

3. Operationsleuchte nach einem der vorhergehenden Ansprüche, wobei die Leuchten (10) und/oder die optischen Abbildungsvorrichtungen (12, 14, 16) nicht dazu eingerichtet sind, zur Veränderung des Lichtfelds mechanisch und/oder elektromechanisch verstellt zu werden.

4. Operationsleuchte nach einem der vorhergehenden Ansprüche, wobei die Mittel (82) zum Abschalten mindestens einer Leuchte (10) dazu eingerichtet sind, jede Leuchte (10) unabhängig voneinander abzuschalten.

5. Operationsleuchte nach einem der vorhergehenden Ansprüche, wobei die Leuchten (10) in der Operationsleuchte so angeordnet sind, dass sie etwa eine Kreisfläche bilden und
die Mittel zum Abschalten (82) mindestens einer Leuchte (10) dazu eingerichtet sind, die am äußeren Rand der Kreisfläche angeordneten Leuchten abzuschalten.

6. Operationsleuchte nach einem der vorhergehenden Ansprüche, wobei die Mittel (84) zum Steuern der Strahlungsleistung dazu eingerichtet sind, die Beleuchtungsstärke auf dem Operationsfeld zu dimmen.

7. Operationsleuchte nach einem der vorhergehenden Ansprüche, wobei die Leuchten (10) LEDs aufweisen und die optischen Abbildungsvorrichtungen (12, 14, 16) Reflektorelemente sind.

8. Operationsleuchte nach einem der vorhergehenden Ansprüche, wobei die Beleuchtungsstärke auf dem Operationsfeld 160 klx nicht übersteigt.

## Claims

1. Operating light comprising
a number of lights (10),
optical imaging devices (12, 14, 16) which are assigned to the lights (10) and configured to direct radiation emitted by the lights (10) onto an operating field and generate an illuminated light field there, and comprising means (82) for switching off at least one of the lights (10),
**characterized in that**
the diameter of the illuminated light field is reduced by switching off the at least one light, with a device (34) being configured to update, in a manner dependent on the at least one light being switched off, the radiant power of the lights (10) that are not switched off in such a way that the illuminance in the light field remains constant.

2. Operating light according to Claim 1, wherein the means for control (84) are configured to control the radiant power of all lights (10) that are not switched off in such a way that the illuminance in the light field remains constant.

3. Operating light according to one of the preceding claims, wherein the lights (10) and/or the optical imaging devices (12, 14, 16) are not configured to be adjusted mechanically and/or electromechanically for the purposes of modifying the light field.

4. Operating light according to one of the preceding claims, wherein the means (82) for switching off at least one light (10) are configured to switch each light (10) off independently of one another.

5. Operating light according to one of the preceding claims, wherein the lights (10) are arranged in the operating light in such a way that they approximately form a circular area and
the means for switching off (82) at least one light (10) are configured to switch off the lights arranged at the outer edge of the circular area.

6. Operating light according to one of the preceding claims, wherein the means (84) for controlling the radiant power are configured to dim the illuminance on the operating field.

7. Operating light according to one of the preceding claims, wherein the lights (10) have LEDs and the optical imaging devices (12, 14, 16) are reflector elements.

8. Operating light according to one of the preceding claims, wherein the illuminance on the operating field does not exceed 160 klx.

## Revendications

1. Lampe chirurgicale comprenant
un nombre donné de lampes (10),
des dispositifs de reproduction (12, 14, 16) optiques associés aux lampes (10), lesquels sont adaptés afin de diriger un rayonnement émis par les lampes (10) dans un champ opératoire et d'y produire un champ lumineux éclairé, et
comprenant
des moyens (82) servant à éteindre au moins une des lampes (10), **caractérisé en ce que**
le diamètre du champ lumineux éclairé est réduit par l'action consistant à éteindre ladite au moins une lampe, dans laquelle un dispositif (34) est adaptés pour régler ultérieurement la puissance de rayonnement des lampes (10) qui n'ont pas été éteintes en fonction de l'opération consistant à éteindre ladite au moins une lampe de sorte que l'intensité d'éclairement reste constante dans le champ lumineux.

2. Lampe chirurgicale selon la revendication 1, dans laquelle les moyens de commande (84) sont adaptés pour commander la puissance de rayonnement de toutes les lampes (10) qui n'ont pas été éteintes de sorte que l'intensité d'éclairement reste constante dans le champ lumineux.

3. Lampe chirurgicale selon l'une quelconque des revendications précédentes, dans laquelle les lampes (10) et/ou les dispositifs de reproduction (12, 14, 16) optiques ne sont pas adaptés pour être ajustés mécaniquement et/ou électromécaniquement aux fins de la variation du champ lumineux.

4. Lampe chirurgicale selon l'une quelconque des revendications précédentes, dans laquelle les moyens (82) servant à éteindre au moins une lampe (10) sont adaptés pour éteindre chaque lampe (10) indépendamment les unes des autres.

5. Lampe chirurgicale selon l'une quelconque des revendications précédentes, dans laquelle les lampes (10) sont disposées dans la lampe chirurgicale de telle sorte qu'elles forment approximativement une surface circulaire, et
les moyens servant à éteindre (82) au moins une lampe (10) sont adaptés afin d'éteindre les lampes disposées au niveau du bord extérieur de la surface circulaire.

6. Lampe chirurgicale selon l'une quelconque des revendications précédentes, dans laquelle les moyens (84) servant à commander la puissance de rayonnement sont adaptés afin d'atténuer l'intensité d'éclairement dans le champ opératoire.

7. Lampe chirurgicale selon l'une quelconque des revendications précédentes, dans laquelle les lampes (10) présentent des DEL et que les dispositifs de reproduction (12, 14, 16) optiques sont des éléments réflecteurs.

8. Lampe chirurgicale selon l'une quelconque des revendications précédentes, dans laquelle l'intensité d'éclairement ne dépasse pas, dans le champ opératoire, 160 klx.
